# EUROPEAN PATENT SPECIFICATION

(11) **EP 3 476 945 B1**
(45) Date of publication and mention of the grant of the patent: **12.10.2022**
(21) Application number: 17831358.1
(22) Date of filing: 20.07.2017
(51) Int. Cl.: C12Q 1/6853, C12Q 1/6848

(54) **MIRNA DETECTING METHOD**
VERFAHREN ZUR MIRNA-DETEKTION
PROCÉDÉ DE DÉTECTION DE MIARN

(30) Priority: 21.07.2016 WO PCT/KR2016/007971
(43) Date of publication of application: 01.05.2019
(73) Proprietor: Heimbiotek Inc., Hwaseong-si, Gyeonggi-do 18384 (KR)
(72) Inventor: LEE, Jae Hoon, Guri-si Gyeonggi-do 11935 (KR)
(74) Representative: Isarpatent
(86) International application number: PCT/KR2017/007804
(87) International publication number: WO 2018/016885

(56) References cited:
- EP-B1- 2 198 059
- CN-A- 103 555 848
- KR-A- 20090 017 670
- KR-A- 20160 098 097
- US-A1- 2013 045 885
- US-A1- 2014 295 434
- US-B2- 8 383 344
- Anonymous: "qPCR Technical Guide", , 2008, XP055648012, Retrieved from the Internet: URL:https://www.gene-quantification.de/SIA L-qPCR-Technical-Guide.pdf [retrieved on 2019-11-29]
- J. R. STULTS ET AL: "Application of the 5' Fluorogenic Exonuclease Assay (TaqMan) for Quantitative Ribosomal DNA and rRNA Analysis in Sediments", APPLIED AND ENVIRONMENTAL MICROBIOLOGY, vol. 67, no. 6, June 2001 (2001-06), pages 2781-2789, XP055648013, US ISSN: 0099-2240, DOI: 10.1128/AEM.67.6.2781-2789.2001

## Description

### TECHNICAL FIELD

The present invention relates to a method for detecting a miRNA molecule.

### BACKGROUND ART

MicroRNA (hereinafter abbreviated as "miRNA") is a noncoding RNA consisting of about 22 ribonucleotides. It is known that miRNA regulates RNA silencing in the transcription stage and gene expression in the post-transcriptional stage. This function of miRNA is performed by base-pairing with a complementary sequence in its targeted mRNA. Since miRNA was first found in *C*. *elegans,* a variety of miRNAs have been reported one after another while it is known that they can regulate gene expression in animals, plants and viruses. In addition, it is known that dysfunctions attributable to mutation of miRNA genes may cause certain diseases such as cancer. Thus, the importance of miRNAs has been more and more recognized.

Since it is known that miRNA is very short in length (about 22 nt) and is more easily degraded than mRNA, there is great difficulty in isolating or detecting miRNA. Conventional methods for detecting miRNA include Northern blot analysis; hybridization-based detection using microarrays; a method of detecting and quantifying a certain miRNA by a two-step process comprising RT-PCR, which uses stem-loop primers binding complementarily to the miRNA, and subsequent quantitative PCR; and a method comprising tailing the 3'-end of miRNA with poly(A) using a poly(A) polymerase, synthesizing cDNA using a poly(T) adaptor as a primer, and then amplifying the miRNA using a miRNA-specific forward primer and a reverse primer based on the poly(T) adaptor.

However, the above-described detection methods have disadvantages in that, because complementary sequences are not enough long, nonspecific amplification may occur, and because these methods are generally based on real-time PCR, expensive real-time PCR systems are required, and because PCR products have similar sizes, multiplex analysis for detecting various miRNAs at the same time is limited.

In addition, in conventional methods for miRNA detection, a reverse transcription primer having a long length of about 70 to 80 mer is used in order to ensure the length of a template for PCR amplification, because the length of miRNA is short. However, in the case of such conventional detection methods, the diffusion of the reverse transcription primer in a reaction solution is slow due to its long length, and thus the reverse transcription reaction time increases, leading to an increase in the overall reaction time. Therefore, the conventional detection methods have problems in that Cₜ values in real-time PCR become greater and reactivity decreases.

US8383344 relates to a method for detecting a miRNA molecule comprising introducing a first and second adaptor into the amplicon, wherein a following PCR is performed using adaptor primers.

CN103555848 relates to a method to detect a miRNA molecule using a tailed reverse transcription primer with about 6-8 bases of the miRNA 3' end and an irrelevant sequence for extension, wherein said primer is likewise used as reverse primer in the subsequent amplification together with a tailed forward primer with an irrelevant sequence for extension.

### SUMMARY OF INVENTION

The present invention has been made in order to solve various problems, including the above-described problems. The present invention provides a method for detecting a miRNA molecule as defined in claims 1 and 2.

### DETAILED DESCRIPTION OF INVENTION

### Definition of Terms

As used herein, the term "oligonucleotide" generally refers to a polymer of nucleotide monomer units, which is a short single-stranded nucleic acid molecule composed of 13 to 25 nucleotides. In some cases, the term may refer to a nucleic acid molecule composed of less than 13 nucleotides, including 6-mer, 7-mer, 8-mer, 9-mer, 10-mer, 11-mer and 12-mer, or more than 25 nucleotides. As used herein, the term "polynucleotide" generally refers to a polymer of nucleotide units, which is longer than the oligonucleotide as defined above, but may also be used interchangeably with the term "oligonucleotide". Polynucleotides include both single-stranded and double-stranded nucleic acid molecules.

As used herein, the term "RNA molecule" refers to a multimeric molecule that performs multiple roles in the coding, decoding, regulation and expression of genes, and generally distinguished from double-stranded DNA in that it forms a single strand, has a hydroxyl group attached to the 2'-carbon of pentose (i.e. ribose) of the nucleic acid unit, and contains uracil instead of thymine in its bases. RNA molecules include mRNA encoding protein, rRNA constituting ribosome, tRNA involved in protein translation, small RNA molecules found within splicing speckles and Cajal bodies of cell nuclei in eukaryotic cells, such as snRNA (small nuclear RNA) functioning to process heterogeneous nuclear RNA (hnRNA), snoRNA (small nucleolar RNA) functioning to guide modification of other RNAs such as rRNA, tRNA, and snRNA, siRNA (small interfering RNA) which is an artificial RNA molecule synthesized to inhibit the expression of a specific gene, exRNA (extracellular RNA or exosomal RNA) present in extracellularly secreted exosomes, piRNA that forms RNA-protein complexes by interacting with piwi protein involved in the epigenetic and post-transcriptional gene silencing of retrotransposons and other genetic elements in germ line cells, and microRNA (or miRNA) which is a small RNA molecule of about 22 nt functioning to regulate the expression of a specific gene.

As used herein, the term "RNA molecule to be amplified" refers to an RNA molecule to be amplified and detected by PCR amplification, among various RNA molecule molecules present in a sample obtained from a subject. This RNA molecule to be amplified may be used as a marker that by its amplification and detection, determines the presence and severity of a certain disease, or sensitivity or resistance to a specific therapeutic agent. As used herein, the term "sense strand" refers to one single-strand nucleic acid molecule of a double-stranded DNA molecule, which is oriented in the same direction as the direction in which the gene of interest is read, and the term "antisense strand" refers to the other single-strand nucleic acid molecule complementary to the sense strand. However, regardless of the direction in which the gene is read, a strand whose nucleic acid sequence is first known may also be defined as "sense strand", and a strand complementary thereto may be defined as "antisense strand".

As used herein, the term "PCR (polymerase chain reaction)" or "nucleic acid amplification reaction" refers to a reaction that amplifies a certain target nucleic acid molecule using a heat-stable DNA polymerase. PCR is performed using a reaction buffer containing, in addition to the DNA polymerase, oligonucleotide primers (forward and reverse primers) capable of hybridizing specifically to a target nucleic acid, a deoxynucleotide (dNTP) mixture, divalent ions such as Mg²⁺, and the like. A DNA molecule produced by the PCR reaction is herein referred to as "amplification product". As used herein, the term "primer extension" refers to a non-chain reaction in which a template nucleic acid having a limited length is extended using a DNA polymerase and primers complementary thereto and which is terminated at the 5'-end of the template nucleic acid. A DNA molecule produced by the primer extension is herein referred to as "extension product". As used herein, the term "primer" refers to an oligonucleotide or polynucleotide that hybridizes complementarily to template DNA and that is used to initiate PCR reaction or primer extension. PCR reaction is performed using a forward primer (or a sense primer) selected from a sense strand which is oriented in the same direction as the direction in which a nucleic acid molecule to be amplified is read, and a reverse primer (or an antisense primer) selected from an antisense strand complementary to the sense strand. Primer extension is generally performed using a single extension primer.

As used herein, the term "adaptor" refers to either an oligonucleotide which is added to the 5'-end of sense DNA corresponding to a certain RNA in order to specifically recognize the RNA, or an oligonucleotide which is attached to the 5'-end of a reverse transcription primer for reverse transcription of RNA. In this case, the adaptor may be used as a primer sequence to amplify extended cDNA, and in the latter case, the adaptor may be used to calibrate a low Tm value which appears when the primer consists only of a nucleic acid sequence capable of hybridizing to RNA. The adaptor may be designed to have no homology to a template nucleic acid to be amplified and other primers, except for an oligonucleotide primer for extension, and thus may be designed so as to minimize nonspecific amplification. As used herein, the term "hybridizing oligonucleotide" refers to a nucleic acid molecule capable of hybridizing complementarily to either RNA or cDNA reverse-transcribed from the RNA. As used herein, the term "universal primer" refers to a primer enabling any kind of nucleic acid to be amplified. The universal primer may be prepared using the nucleic acid sequence of an adaptor oligonucleotide added for reverse transcription and primer extension. In this case, there is an advantage in that a single primer set may be used, thereby reducing the production cost of the kit.

As used herein, the term "forward primer" refers to a primer oriented in the same direction in which the gene of interest is read (5'-to-3' direction). On the contrary, the term "reverse primer" refers to a primer oriented opposite to a direction in which the gene of interest is read.

As used herein, the term "Cₜ value (threshold cycle)" refers to the cycle number of real-time PCR for measuring an amplification product in real time within the range of exponential phase, at the time the intensity of the fluorescence measured significantly increases to the extent that it is sensible beyond the baseline level in the real-time PCR. It reflects the amount of initial template of a target RNA.

### Disclosure of the Application

The following disclosure assists the understanding of the present invention. The present disclosure provides a kit and method for detecting RNA, which enables fast diffusion of a reverse transcription primer in a reaction solution by adopting a short reverse transcription primer in a reverse transcription process, and thus can shorten the reverse transcription reaction time. In this connection, the present disclosure makes it possible to increase the overall PCR reaction rate and to detect a target RNA stably and quickly. The present disclosure also provides a kit and a method for detecting RNA, which can ensure a sufficient template length for PCR amplification by performing an extension reaction, using an extension primer much longer than the reverse transcription primer, on a single-stranded cDNA obtained after the reverse transcription process.

In accordance with one aspect of the present disclosure, there is provided a kit for detecting an RNA molecule, comprising:
a short reverse transcription primer composed of a first hybridizing oligonucleotide and a first adaptor oligonucleotide, the first hybridizing oligonucleotide being composed of an oligo(dT) which hybridizes to a poly(A) tail of an RNA molecule in s sample, and the first adaptor oligonucleotide being attached to the 5'-end of the first hybridizing oligonucleotide and being any nucleic acid sequence which does not hybridize to the RNA molecule;
an extension primer longer than the reverse transcription primer and composed of a second hybridizing oligonucleotide and a second adaptor oligonucleotide, the second hybridizing oligonucleotide being capable of hybridizing specifically to a 3'-end portion of a single-stranded cDNA reverse-transcribed from the RNA molecule, the portion of the single-stranded cDNA excluding the oligo(dT), and the second adaptor oligonucleotide being attached to the 5'-end of the second hybridizing oligonucleotide and having any nucleic acid sequence which does not hybridize to the single-stranded cDNA; and
a forward primer having a sequence within the sequence of the second adaptor oligonucleotide.

In the kit, the RNA molecule may be a small RNA molecule which is 18 to 180 nt, 20 to 90 nt, 20 to 70 nt, 20 to 50 nt, or 20 to 30 nt in length. The small RNA molecule may be tRNA, snRNA, siRNA, exRNA, piRNA, scaRNA or miRNA. In the kit, the sample may be epidermis, hair, biopsy, autopsy, blood, plasma, serum, saliva, urine, feces, sweat, phlegm, nasal discharge, or tear.

The kit may further comprise a reverse primer having a nucleic acid sequence within the first adaptor oligonucleotide. The reverse primer may be a universal reverse primer having a constant nucleic acid sequence regardless of the kind of RNA molecule to be amplified.

However, in a disclosure, the kit does not comprise the additional reverse primer. In this case, the short reverse transcription primer used in the reverse transcription reaction also functions as a reverse primer in PCR reaction. Even when the reverse primer is not added in PCR reaction, the short reverse transcription primer contained in the reverse transcription reactant may also be used in PCR reaction as it remains therein.

In the kit, the second adaptor oligonucleotide may comprise either an RNA-tagging oligonucleotide dependent on the kind of RNA molecule to be amplified, or the RNA-tagging oligonucleotide and a consensus oligonucleotide having a consensus nucleic acid sequence independent of the kind of RNA to be amplified, and the forward primer may be a universal forward primer comprising a nucleic acid sequence selected from the consensus oligonucleotide.

In the kit, the short reverse transcription primer may be about 29 to 34 nt in length, and the first adaptor oligonucleotide may be about 17 to 22 nt in length. In the kit, the second adaptor oligonucleotide may be about 32 to 61 nt or about 56 to 63 nt in length, and the length thereof is adjustable depending on the kind of RNA molecule to be amplified. In this case, two or more RNAs to be amplified may be multiplex-detected in a single reaction. In the kit, the second hybridizing oligonucleotide may be 12 to 22 nt, 13 to 22 nt, or 14 to 22 nt in length, and does not hybridize to the short reverse transcription primer.

In accordance with another aspect of the present disclosure, there is provided a kit for detecting an RNA molecule, comprising:
a short reverse primer for reverse transcription composed of a first hybridizing oligonucleotide and a first adaptor oligonucleotide, the first hybridizing oligonucleotide being composed of a nucleic acid sequence which hybridizes to a 3'-end portion of the RNA molecule having 5 to 9 nt at the 3'-end of the RNA molecule, and the first adaptor oligonucleotide being attached to the 5'-end of the first hybridizing oligonucleotide and being any nucleic acid sequence which does not hybridize to the RNA molecule;
an extension primer longer than the reverse primer for reverse transcription and composed of a second hybridizing oligonucleotide and a second adaptor oligonucleotide, the second hybridizing oligonucleotide being capable of hybridizing specifically to a portion of a single-stranded cDNA reverse-transcribed from the RNA molecule, the portion of the single-stranded cDNA excluding a portion corresponding to the 3'-end portion of the RNA molecule, and the second adaptor oligonucleotide being attached to the 5'-end of the second hybridizing oligonucleotide and having any nucleic acid sequence which does not hybridize to the single-stranded cDNA; and
a forward primer having a sequence within the sequence of the second adaptor oligonucleotide.

In the kit, the RNA molecule may be a small RNA molecule which is 18 to 180 nt, 20 to 90 nt, 20 to 70 nt, 20 to 50 nt, or 20 to 30 nt in length. The small RNA molecule may be tRNA, snRNA, siRNA, exRNA, piRNA, scaRNA or miRNA.

The kit may further comprise a reverse primer having a nucleic acid sequence within the first adaptor oligonucleotide. The reverse primer may be a universal reverse primer having a constant nucleic acid sequence regardless of the kind of RNA molecule to be amplified. However, in a disclosure, the kit does not comprise the additional reverse primer. In this case, the short reverse primer for reverse transcription used in the reverse transcription reaction also functions as a reverse primer in PCR reaction. Even when the reverse primer is not added in PCR reaction, the short reverse primer for reverse transcription contained in the reverse transcription reactant may also be used in PCR reaction as it remains therein.

In the kit, the second adaptor oligonucleotide may comprise either an RNA-tagging oligonucleotide dependent on the kind of RNA molecule to be amplified, or the RNA-tagging oligonucleotide and a consensus oligonucleotide having a consensus nucleic acid sequence independent of the kind of RNA to be amplified, and the forward primer may be a universal forward primer comprising a nucleic acid sequence selected from the consensus oligonucleotide.

In the kit, the short reverse primer for reverse transcription may be about 26 to 28 nt, 23 to 28 nt, or 18 to 28 nt in length, and the first adaptor oligonucleotide may be about 20 to 22 nt, 17 to 22 nt, or 12 to 22 nt in length. In the kit, the second adaptor oligonucleotide may be about 32 to 61 nt or about 56 to 63 nt in length, and the length thereof is adjustable depending on the kind of RNA molecule to be amplified. In this case, two or more RNAs to be amplified may be multiplex-detected in a single reaction.

In the kit, the first hybridizing oligonucleotide may be 6 to 8 nt in length, more preferably 6 to 7 nt in length, and most preferably 6 nt in length. The second hybridizing oligonucleotide may be 12 to 16 nt, 13 to 16 nt, or 14 to 16 nt in length, and does not hybridize to the short reverse primer for reverse transcription.

In accordance with still another aspect of the present disclosure, there is provided a method for detecting an RNA molecule, comprising:
tailing an RNA molecule in a sample obtained from a subject, with poly(A) by using a poly(A) polymerase, thereby preparing a poly(A)-tailed RNA molecule;
reverse-transcribing the poly(A)-tailed RNA molecule using a reverse transcriptase and a short reverse transcription primer composed of a first hybridizing oligonucleotide and a first adaptor oligonucleotide, the first hybridizing oligonucleotide being composed of an oligo(dT) which hybridizes to the poly(A) tail of the poly(A)-tailed RNA molecule, and the first adaptor oligonucleotide being attached to the 5'-end of the first hybridizing oligonucleotide and being any nucleic acid sequence which does not hybridize to the poly(A)-tailed RNA molecule;
deactivating the reverse transcriptase and melting a DNA produced by the reverse transcription, thereby preparing a single-stranded cDNA reverse-transcribed from the poly(A)-tailed RNA molecule; and
transferring a portion or all of the reverse transcription reactant into a reactor for primer extension and PCR reactions containing an extension primer longer than the reverse transcription primer, a forward primer, and a heat-stable DNA polymerase, the extension primer being composed of a second hybridizing oligonucleotide and a second adaptor oligonucleotide, the second hybridizing oligonucleotide being capable of hybridizing specifically to a 3'-end portion of the single-stranded cDNA, the portion of the single-stranded cDNA excluding the oligo(dT), and the second adaptor oligonucleotide being attached to the 5'-end of the second hybridizing oligonucleotide and having any nucleic acid sequence which does not hybridize to the single-stranded cDNA, and the forward primer having a sequence within the second adaptor oligonucleotide, and then performing primer extension and PCR reactions in a single step.

In the detection method, the RNA molecule may be a small RNA molecule which is 18 to 180 nt, 20 to 90 nt, 20 to 70 nt, 20 to 50 nt, or 20 to 30 nt in length. The small RNA molecule may be tRNA, snRNA, siRNA, exRNA, piRNA, scaRNA or miRNA. In the detection method, the sample may be epidermis, hair, biopsy, autopsy, blood, plasma, serum, saliva, urine, feces, sweat, phlegm, nasal discharge, or tear.

In the detection method, a reverse primer having a nucleic acid sequence within the first adaptor oligonucleotide may additionally be used in the PCR reaction. The reverse primer may be a universal reverse primer having a constant nucleic acid sequence regardless of the kind of RNA molecule to be amplified. However, in a disclosure, the additional reverse primer is not used, and in this case, the short reverse transcription primer used in the reverse transcription reaction also functions as a reverse primer in the PCR reaction. Even when the reverse primer is not added in the PCR reaction, the short reverse transcription primer contained in the reverse transcription reactant may also be used in the PCR reaction as it remains therein. The present inventor has found that undesirable results such as the appearance of non-specific bands were obtained when the reverse primer was additionally added in the PCR reaction, whereas more desirable results were obtained when the reverse primer was not added in the PCR reaction.

In the detection method, the second adaptor oligonucleotide may comprise either an RNA-tagging oligonucleotide dependent on the kind of RNA molecule to be amplified, or the RNA-tagging oligonucleotide and a consensus oligonucleotide having a consensus nucleic acid sequence independent of the kind of RNA to be amplified, and the forward primer may be a universal forward primer comprising a nucleic acid sequence selected from the consensus oligonucleotide.

In the detection method, the short reverse transcription primer may be about 29 to 34 nt in length, and the first adaptor oligonucleotide may be about 17 to 22 nt in length. In the detection method, the second adaptor oligonucleotide may be about 32 to 61 nt or about 56 to 63 nt in length, and the length thereof is adjustable depending on the kind of RNA molecule to be amplified. In this case, two or more RNAs to be amplified may be multiplex-detected in a single reaction. In the detection method, the second hybridizing oligonucleotide may be 12 to 22 nt, 13 to 22 nt, or 14 to 22 nt in length, and does not hybridize to the short reverse transcription primer.

In accordance with still another aspect of the present disclosure, there is provided a method for detecting an RNA molecule, comprising:
reverse-transcribing an RNA molecule in a sample using a reverse transcriptase and a short reverse primer for reverse transcription composed of a first hybridizing oligonucleotide and a first adaptor oligonucleotide, the first hybridizing oligonucleotide being composed of a nucleic acid sequence which hybridizes to a 3'-end portion of the RNA molecule having 5 to 9 nt at the 3'-end of the RNA molecule, and the first adaptor oligonucleotide being attached to the 5'-end of the first hybridizing oligonucleotide and being any nucleic acid sequence which does not hybridize to the RNA molecule;
deactivating the reverse transcriptase and melting a DNA produced by the reverse transcription, thereby preparing a single-stranded cDNA reverse-transcribed from the RNA molecule; and
transferring a portion or all of the reverse-transcription reactant into a reactor for primer extension and PCR reactions containing an extension primer longer than the reverse primer for reverse transcription, a forward primer, and a heat-stable DNA polymerase, the extension primer being composed of a second hybridizing oligonucleotide and a second adaptor oligonucleotide, the second hybridizing oligonucleotide being capable of hybridizing specifically to a portion of the single-stranded cDNA reverse-transcribed from the RNA molecule, the portion of the single-stranded cDNA excluding a portion corresponding to the 3'-end portion of the RNA molecule, and the second adaptor oligonucleotide being attached to the 5'-end of the second hybridizing oligonucleotide and having any nucleic acid sequence which does not hybridize to the single-stranded cDNA, and the forward primer having a sequence within the second adaptor oligonucleotide, and then performing primer extension and PCR reactions in a single step.

In the detection method, the RNA molecule may be a small RNA molecule which is 18 to 180 nt, 20 to 90 nt, 20 to 70 nt, 20 to 50 nt, or 20 to 30 nt in length. The small RNA molecule may be tRNA, snRNA, siRNA, exRNA, piRNA, scaRNA or miRNA. In the detection method, the sample may be epidermis, hair, biopsy, autopsy, blood, plasma, serum, saliva, urine, feces, sweat, phlegm, nasal discharge, or tear.

The detection method, a reverse primer having a nucleic acid sequence within the first adaptor oligonucleotide may additionally be used. The reverse primer may be a universal reverse primer having a constant nucleic acid sequence regardless of the kind of RNA molecule to be amplified. However, in a disclosure, the kit does not comprise the additional reverse primer. In this case, the short reverse primer for reverse transcription used in the reverse transcription reaction also functions as a reverse primer in the PCR reaction. Even when the reverse primer is not added in the PCR reaction, the short reverse primer for reverse transcription contained in the reverse transcription reactant may also be used in the PCR reaction as it remains therein. The present inventor has found that undesirable results such as the appearance of non-specific bands were obtained when the reverse primer was additionally added in the PCR reaction, whereas more desirable results were obtained when the reverse primer was not added in the PCR reaction.

In the detection method, the second adaptor oligonucleotide may comprise either an RNA-tagging oligonucleotide dependent on the kind of RNA molecule to be amplified, or the RNA-tagging oligonucleotide and a consensus oligonucleotide having a consensus nucleic acid sequence independent of the kind of RNA to be amplified, and the forward primer may be a universal forward primer comprising a nucleic acid sequence selected from the consensus oligonucleotide.

In the detection method, the short reverse primer for reverse transcription may be about 26 to 28 nt, 23 to 28 nt, or 18 to 28 nt in length, and the first adaptor oligonucleotide may be about 20 to 22 nt, 17 to 22 nt, or 12 to 22 nt in length. In the detection method, the second adaptor oligonucleotide may be about 32 to 61 nt or about 56 to 63 nt in length, and the length thereof may is adjustable depending on the kind of RNA molecule to be amplified. In this case, two or more RNAs to be amplified may be multiplex-detected in a single reaction.

In the detection method, the first hybridizing oligonucleotide may be 6 to 8 nt in length, more preferably 6 to 7 nt in length, and most preferably 6 nt in length. The second hybridizing oligonucleotide may be 12 to 16 nt, 13 to 16 nt, or 14 to 16 nt in length, and does not hybridize to the short reverse primer for reverse transcription.

Unless otherwise specified herein, a nucleic acid molecule composed of a plurality of oligonucleotides and/or polynucleotides is read from the 5'-end to the 3'-end.

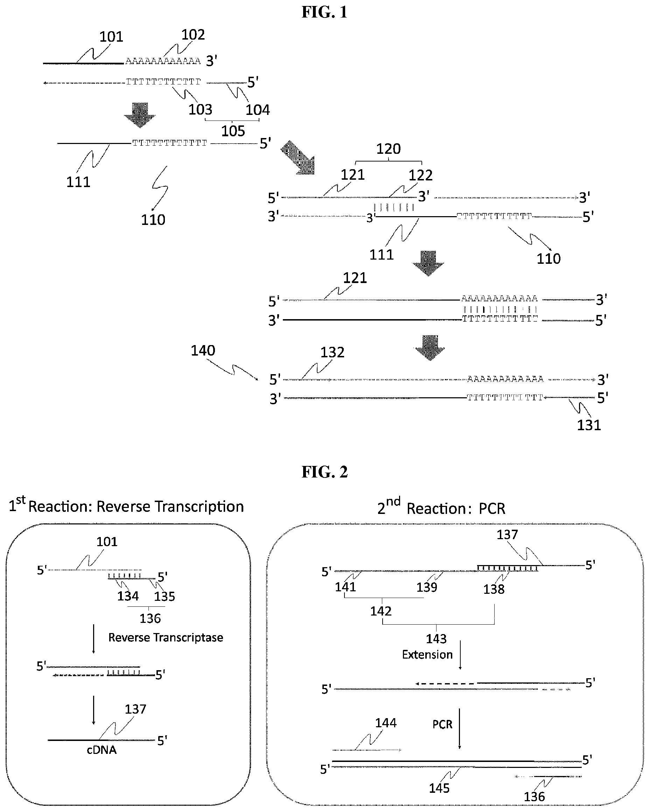

FIG. 1 is a schematic view illustrating a method of detecting a small RNA using a poly(A) polymerase according to a disclosure. As shown in FIG. 1, an exemplified RNA molecule 101 may be a very short nucleic acid molecule, such as miRNA, which is about 20 to 30 nt in length. The miRNA 101 has no poly(A) tail, unlike mRNA. For this reason, when a poly(A) polymerase and ATP are mixed and reacted with a sample containing the miRNA 101, a poly(A) tail 102 is then attached to the miRNA 101 to produce a poly(A)-tailed miRNA (first reaction). When a reverse transcription primer 105, composed of a first adaptor oligonucleotide 104 having a nucleic acid which does not hybridize to an RNA molecule to be amplified, and a poly dT which is a first hybridizing oligonucleotide 103, is added to the poly(A)-tailed miRNA, the first hybridizing oligonucleotide 103 then binds complementarily to the poly(A) tail. When a reverse transcriptase and a dNTP mixture is added to the miRNA complex and a reverse transcription reaction is performed, a reverse transcription product 110 of the target miRNA is produced which consists of the first adaptor oligonucleotide 104, the first hybridizing oligonucleotide 103 and an antisense strand cDNA 111 (second reaction).

Then, the reverse transcriptase is deactivated and the DNA is melted, thereby ensuring that the reverse transcription process is terminated, thereby preparing a single-stranded cDNA. Then, an extension reaction is performed using an extension primer much longer than the reverse transcription primer in order to ensure a sufficient template length for PCR amplification. However, the extension reaction is performed on the single-stranded cDNA, not heteroduplex cDNA, by use of an extension primer much longer than the reverse transcription primer, in order to prevent the decrease of reactivity of the extension primer.

A portion or all of the reverse transcription product 110 is transferred to a second reactor, and hybridized to an extension primer 120 (longer than the reverse transcription primer), which has at the 3'-end a second hybridizing oligonucleotide 122 having a sequence complementary to the antisense strand cDNA and at the 5'-end a second adaptor oligonucleotide 121 having any nucleic acid sequence that does not hybridize to both of the miRNA 110 and the antisense strand cDNA 111. Then, primer extension with a DNA polymerase is performed, thereby producing an extended double-stranded cDNA 140 (third reaction). Since the length of the second adaptor oligonucleotide can be adjusted according to each target RNA, each of target RNAs can be labeled by the different length of its respective second adaptor oligonucleotide, making it possible to detect a plurality of RNAs by multiplex analysis.

Using the extended double-stranded cDNA 140 as a template, a PCR reaction is performed using a reverse primer 131 having a nucleic acid sequence identical to that of the first adaptor oligonucleotide and a forward primer 132 having a nucleic acid sequence of the 5'-end of the second adaptor oligonucleotide, thereby exponentially amplifying the extended double-stranded cDNA 140 (fourth reaction). The primer extension reaction (third reaction) and the PCR reaction (fourth reaction) may not be sequentially performed, but may be performed in a single reaction. In addition, the reverse transcription primer 105 used in the second reaction may be used instead of the reverse primer 131. In this case, the reverse transcription primer 105 remained in the second reaction product when transferring a portion or all of the second reaction product into the reactor for primer extension and PCR reactions may be reused in the fourth reaction, rather than separately adding the reverse transcription primer 105 in the fourth reaction.

FIG. 2 is a schematic view illustrating a method of detecting a miRNA molecule using an RNA-specific primer (reverse transcription primer) according to one embodiment of the present invention. As shown in FIG. 2, an RNA-specific reverse primer 136 (short reverse transcription primer), which has at the 3'-end a first hybridizing oligonucleotide 134 capable of specifically binding to a 5-7-nt (preferably 6-nt) portion of the 3'-end of an RNA 101, and at the 5'-end a first adaptor oligonucleotide 135 linked to the first hybridizing oligonucleotide and having any nucleic acid sequence that does not hybridize to a target RNA, is hybridized to the RNA, and then is subjected to reverse transcription using a reverse transcriptase, thereby producing an antisense strand cDNA 137 (first step reaction).

The reverse transcriptase is deactivated and the DNA is melted, ensuring that the reverse transcription process is terminated, thereby preparing a single-stranded cDNA. Then, an extension reaction is performed using an extension primer much longer than the reverse transcription primer in order to ensure a sufficient template length for PCR amplification. However, the extension reaction is performed on the single-stranded cDNA, not heteroduplex cDNA, by use of the extension primer much longer than the reverse transcription primer, in order to prevent the decrease of reactivity of the extension primer.

When the antisense strand cDNA 137 is subjected to primer extension using an extension primer 143 which has at the 3'-end a second hybridizing oligonucleotide 138 capable of specifically binding to one portion (about 16 nt in length) of the antisense strand cDNA 137 excluding the other portion corresponding to the aforesaid 6 nt portion of the RNA 101, and at the 5'end a second adaptor oligonucleotide 142 which is linked to the second hybridizing oligonucleotide 138 and which does not bind complementarily to both of the antisense strand cDNA 137 and the RNA 101, the cDNA is then extended in both directions, thereby producing an extension product comprising strands complementary to the RNA-specific primer 136 and to the second adaptor oligonucleotide 142. Here, the second adaptor oligonucleotide 142 comprises at its 5'-end a 5'-region 141 that is an universal primer region, which is used commonly in all RNA detection kits, and the remaining 3'-region 139 of the second adaptor oligonucleotide 142 may have different sequences and/or lengths depending on the kind of RNA, thereby enabling various RNAs to be detected at the same time by multiplex analysis. After completion of the primer extension reaction, a PCR product 145 is produced in the same tube by PCR using the RNA-specific primer 136 and a forward primer 144 corresponding to the 5'-region 141 (second step reaction).

The forward primer 144 has a sequence within the nucleic acid sequence of the 5'-end of the second adaptor oligonucleotide. If the sequence of the 5'-end of the second adaptor oligonucleotide is designed to be constant regardless of the kind of RNA, the forward primer 144 becomes a universal forward primer. The reverse transcription primer in the reverse transcription reaction can be used as the RNA-specific primer 136. The reverse transcription primer may further be added in the second step reaction, or the reverse transcription primer remained in the product of the first step reaction may also be used in the second step reaction without further adding the reverse transcription primer. The PCR product 145 can be detected by real-time PCR during the PCR reaction, and can be visualized by agarose gel electrophoresis or the like after completion of the PCR reaction.

### ADVANTAGEOUS EFFECTS

A method for detecting a miRNA molecule according to one embodiment of the present invention uses a short reverse transcription primer, and thus can shorten the reverse transcription reaction time. In this connection, the method of the present invention make it possible to increase the overall PCR reaction rate and to detect RNA quickly. Further, the method can prepare a template having a sufficient length for PCR amplification by using an extension primer much longer than the reverse transcription primer in a primer extension step. Specifically, the method enables fast diffusion of a reverse transcription primer in a reaction solution by adopting a short reverse transcription primer in a reverse transcription process, and thus can shorten the reverse transcription reaction time. In this connection, the method makes it possible to increase the overall PCR reaction rate, lower the Cₜ value and detect RNA stably and quickly. In addition, the method can ensure a sufficient template length for PCR amplification by performing an extension reaction, using an extension primer much longer than the reverse transcription primer, on a single-stranded cDNA obtained after the reverse transcription process.

Furthermore, the method shows high accuracy for detecting a miRNA moleculesince a cDNA produced in a reverse transcription step is further verified in a real-time PCR step, and then a final amplification product is synthesized. Moreover, according to the method, each target miRNA moleculecan be labeled by adjusting the length of the long extension primer. Therefore, the method can detect a certain miRNA in a quick and accurate manner without using an expensive equipment, and can perform multiplex analysis capable of detecting a plurality of miRNAs at the same time. For example, the method can be very effectively used to diagnose various diseases, including cancer, and determine prognosis of such diseases.

It should be understood that the scope of the present is not limited by the aforementioned effects.

### BRIEF DESCRIPTION OF DRAWINGS

The above and other objects and features of the present invention will be clearly understood by those skilled in the art from the description of embodiments of the present invention with reference to the following drawings.
FIG. 1 is a schematic view illustrating a method of detecting miRNA using a poly(A) polymerase according to a disclosure.
FIG. 2 is a schematic view illustrating a method of detecting miRNA using a miRNA-specific reverse transcription primer according to one embodiment of the present invention.

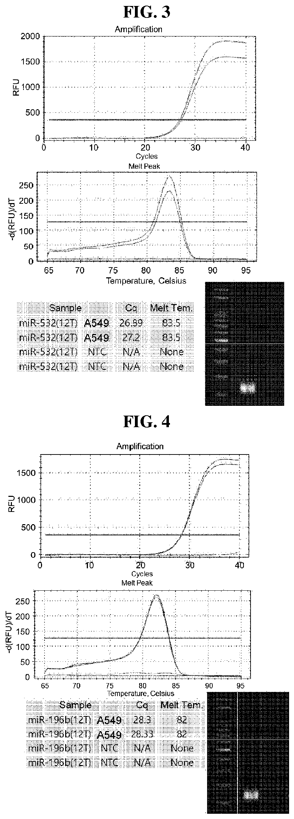

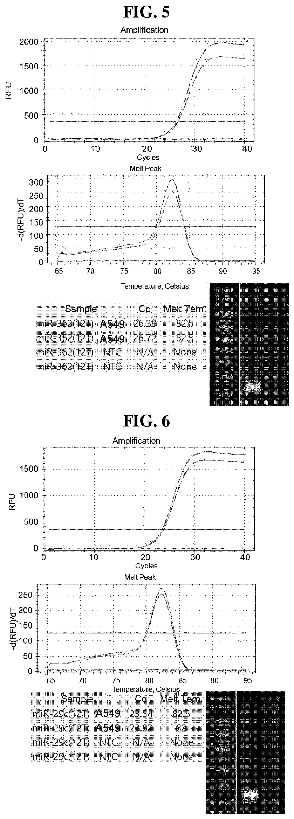

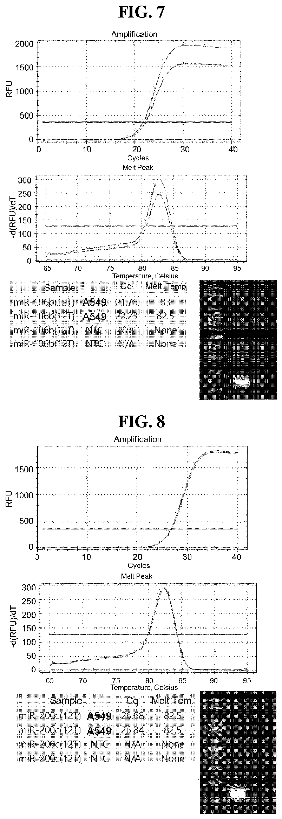

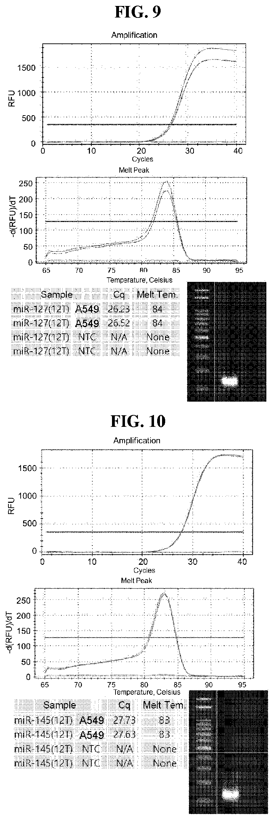

FIGS. 3 to 10 show the results of amplifying miR-532, miR-196b, miR-362, miR-29c, miR-106b, miR-200c, miR-127 and miR-145, respectively, by a miRNA detection kit and method using a poly(A) polymerase and oligo dT primers of a disclosure. In each of FIGS. 3 to 10, the top is a graph showing the change in fluorescence intensity as a function of the number of amplification cycles; the middle is a graph showing the change in fluorescence emission rate as a function of temperature; the table on the left side of the bottom shows the kind of sample, the threshold for fluorescence detection, and melting point; and the right side of the bottom is a photograph showing the result of 1.5% agarose gel electrophoresis.

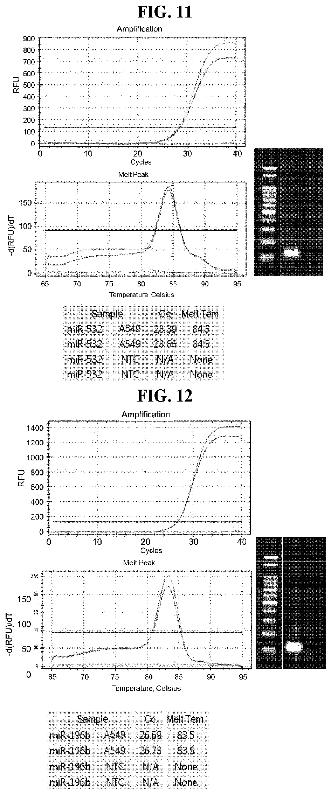

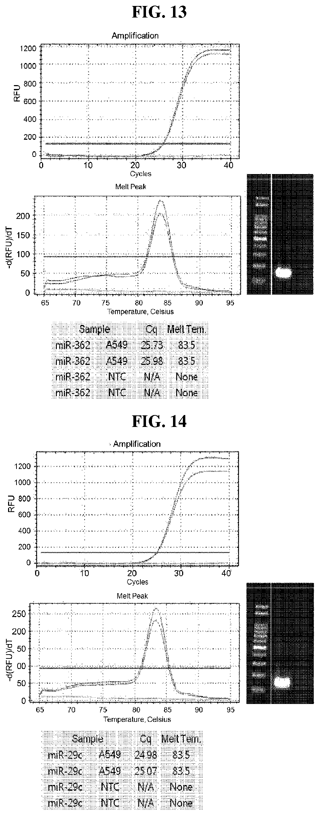

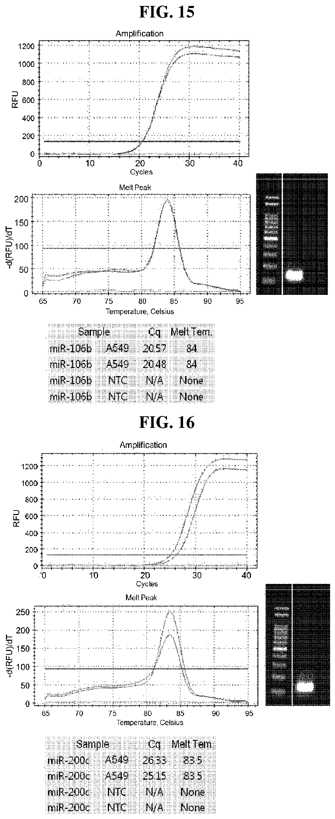

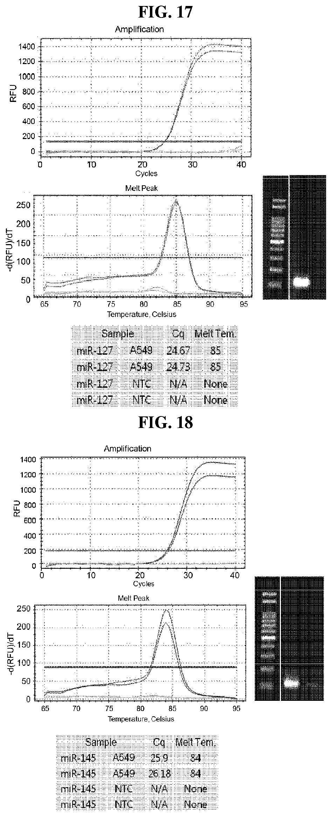

FIGS. 11 to 18 show the results of amplifying miR-532, miR-196b, miR-362, miR-29c, miR-106b, miR-200c, miR-127 and miR-145, respectively, by a miRNA detection method using miRNA-specific reverse transcription primers in examples of the present invention. In each of FIGS. 11 to 18, the top of the left side is a graph showing the change in fluorescence intensity as a function of the number of amplification cycles; the middle of the left side is a graph showing the change in fluorescence emission rate as a function of temperature; the table on the bottom of the left side shows the kind of sample, the threshold for fluorescence detection, and melting point; and the right side is a photograph showing the result of 1.5% agarose gel electrophoresis.

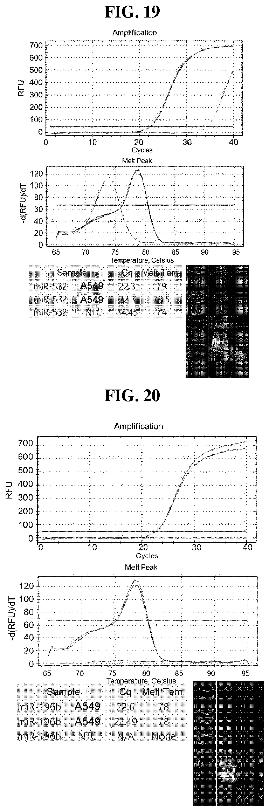

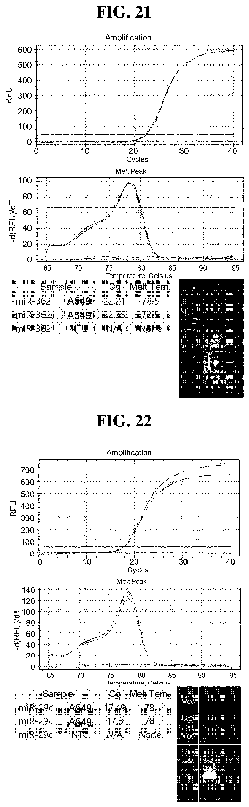

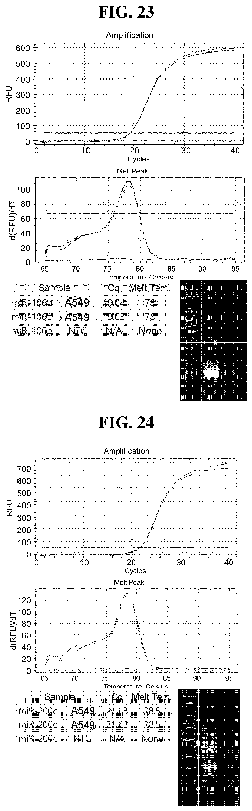

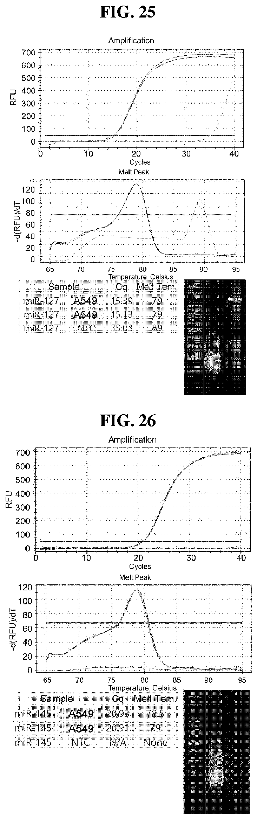

FIGS. 19 to 26 show the results of amplifying miR-532, miR-196b, miR-362, miR-29c, miR-106b, miR-200c, miR-127 and miR-145, respectively, by use of a miRNA detection kit (Qiagen) in comparative examples. In each of FIGS. 19 to 26, the top is a graph showing the change in fluorescence intensity as a function of the number of amplification cycles; the middle is a graph showing the change in fluorescence emission rate as a function of temperature; the table on the left side of the bottom shows the kind of sample, the threshold for fluorescence detection, and melting point; and the right side of the bottom is a photograph showing the result of 1.5% agarose gel electrophoresis.
FIG. 27 is a graph showing the results of performing real-time PCR amplification of miRNA let-7e using each of a miRNA detection kit according to one embodiment of the present invention and a commercial miRNA detection kit (Qiagen).
FIG. 28 is a graph showing the results of sequencing PCR amplification products obtained by performing real-time PCR of miRNA hsa let-7e using each of a miRNA detection method according to one embodiment of the present invention and a commercial miRNA detection kit (Qiagen).
FIG. 29 is a graph showing the correlation between template copy number and detection threshold (C_{q}) cycles in the results of performing real-time PCR on serially diluted miRNAs as templates, using a miRNA detection kit according to one embodiment of the present invention.
FIG. 30 is a graph showing the results of quantitatively measuring the expression levels of three subtypes of miR-200 (miR-200a, miR-200b and miR-200c) in CAL27 cells and HSC3 cells by a miRNA detection method according to one embodiment of the present invention.
FIG. 31 is a set of graphs showing real-time PCR results of a miRNA detection method according to one embodiment of the present invention, depending on whether or not the reverse transcription primer is added in the primer extension and real-time PCR reactions (second step reaction).
FIG. 32 is a set of graphs comparing Cₜ values obtained using a miRNA detection method according to one embodiment of the present invention with Cₜ values obtained using a commercial miRNA detection kit (Qiagen).

### Examples

Hereinafter, the present invention will be described in detail with reference to examples and experimental examples. However, the present invention may be embodied in different forms and should not be construed as limited to the examples disclosed below. Rather, these examples are provided so that this disclosure will be thorough and complete, and will fully convey the scope of the present invention to those skilled in the art. Therefore, it should be understood that these examples and experimental examples are provided merely to explain the present invention in detail and are not intended to restrict or limit the scope of the present invention.

### Examples and Reference Examples: Detection of a miRNA

According to one disclosure, the present inventor detected various miRNAs by tailing each of miRNAs with poly(A), and then performing reverse transcription using an oligo dT primer, and performing bidirectional primer extension and real-time PCR using an extension primer having miRNA-specific sequence, on the reverse transcription product containing the reverse-transcribed cDNA in a single step. According to an embodiment of the present invention, the present inventor detected various miRNAs using a miRNA-specific primer (short reverse transcription primer), which has a 6-nt nucleic acid sequence specific for the 3'-end of miRNA, and an extension primer (longer than the reverse transcription primer) having a miRNA-specific sequence other than the 6-nt nucleic acid sequence, without using an oligo(dT) primer. The miRNAs detected are shown in Table 1 below.

In the detection method performed using the poly(A) polymerase, poly(A) tailing and reverse transcription were performed at 42°C for 60 minutes, and then the reverse transcriptase was deactivated by heating at 70°C for 10 minutes. Primer extension and PCR were performed by melting DNA at 95°C for 15 minutes, and then performing 40 cycles, each consisting of 94°C for 15 sec, 55°C for 30 sec, and 70°C for 30 sec. Next, a melting curve was measured by heating at 95°C for 10 sec, and then heating from 65°C to 95°C at a rate of 0.5°C per 5 sec.

In the detection method performed using the miRNA-specific primer, reverse transcription was performed at 42°C for 60 minutes, and then the reverse transcriptase was deactivated by heating at 70°C for 10 minutes. Primer extension and PCR were performed by melting DNA at 95°C for 15 minutes, and then performing 40 cycles, each consisting of 95°C for 10 sec and 60°C for 40 sec. Next, a melting curve was measured by heating at 95°C for 10 sec, and then heating from 65°C to 95°C at a rate of 0.5°C per 5 sec.

In addition, the amounts of reagents used in the reverse transcription and real-time PCR reactions are shown in Tables 2 and 3 below.

The PCR reaction was performed using a Bio-Rad real-time PCR system (Bio-Rad CFX96 Touch Real-time PCR system), and whether or not the PCR reaction would be actually achieved was analyzed using 1.5% agarose gel electrophoresis band patterns.

**Table 1: Examples of the present invention (9-16) and reference examples (1-8)**

| Example | miRNA to be amplified | Use of poly(A) polymerase | Reverse transcription primer (SEQ ID NO) | Extension primer (SEQ ID NO) | Forward primer | Reverse primer |
|---|---|---|---|---|---|---|
| 1 | miR-532 | Y | 1 | 10 | 18 | 19 |
| 2 | miR-196b | Y | | 11 | | |
| 3 | miR-362 | Y | | 12 | | |
| 4 | miR-29c | Y | | 13 | | |
| 5 | miR-106b | Y | | 14 | | |
| 6 | miR-200c | Y | | 15 | | |
| 7 | miR-127 | Y | | 16 | | |
| 8 | miR-145 | Y | | 17 | | |
| 9 | miR-532 | N | 2 | 10 | | 2 |
| 10 | miR-196b | N | 3 | 11 | | 3 |
| 11 | miR-362 | N | 4 | 12 | | 4 |
| 12 | miR-29c | N | 5 | 13 | | 5 |
| 13 | miR-106b | N | 6 | 14 | | 6 |
| 14 | miR-200c | N | 7 | 15 | | 7 |
| 15 | miR-127 | N | 8 | 16 | | 8 |
| 16 | miR-145 | N | 9 | 17 | | 9 |

**Table 2: Amounts of reagents used in reverse transcription**

| Sample | Volume | Final concentration |
|---|---|---|
| 2X reaction buffer | 10 µL | |
| Reverse transcriptase (AddScript) | 1 µL | 1 U |
| dNTP mix (10 mM) | 2 µL | 1 mM |
| Reverse transcription primer (80 µM) | 1 µL | 4 µM |
| Template (total RNA from A549 cell, 1 µg/µL) | 1 µL | 1 µg |
| Distilled water | 5 µL | |
| Total volume | 20 µL | |

**Table 3: Amounts of reagents used in real-time PCR**

| Sample | Volume | Final concentration |
|---|---|---|
| 2X SYBR Green PCR Master Mix | 10 µL | 1X |
| Extension primer (200 nM) | 1 µL | 10 nM |
| Forward primer (2 µM) | 1 µL | 100 nM |
| Reverse transcription reaction product | 1 µL | |
| Distilled water | 7 µL | |
| Total volume | 20 µL | |

### Comparative Examples

In comparative examples, the present inventor amplified miRNAs using a commercial miRNA detection kit (Qiagen) in order to compare the performance of the miRNA detection kit of the present invention with that of the commercial miRNA detection kit (Qiagen).

**Table 4: Comparative Examples**

| Comparative Example | miRNA to be amplified | Detection method |
|---|---|---|
| 1 | miR-532 | Use of anchor oligo dT adaptor |
| 2 | miR-196b | Use of anchor oligo dT adaptor |
| 3 | miR-362 | Use of anchor oligo dT adaptor |
| 4 | miR-29c | Use of anchor oligo dT adaptor |
| 5 | miR-106b | Use of anchor oligo dT adaptor |
| 6 | miR-200c | Use of anchor oligo dT adaptor |
| 7 | miR-127 | Use of anchor oligo dT adaptor |
| 8 | miR-145 | Use of anchor oligo dT adaptor |

The analysis results are shown in FIGS. 3 to 26. As can be seen therein, the miRNA detection method according to the embodiments of the present invention enabled miRNAs to be detected more clearly and accurately than the commercial miRNA detection kit (Qiagen) (FIGS. 19 to 26).

### Experimental Example 1: Sequencing of PCR Amplification Products

In this experimental example, miRNA hsa let-7e from A549 cells was amplified using the method of the present invention that does not use poly(A) polymerase among the aforesaid methods of the embodiments, and the commercial miRNA detection kit as a comparative example. The PCR amplification products were sequenced.

Specifically, reverse transcription and real-time PCR were performed using a reverse transcription primer having a nucleic acid sequence represented by SEQ ID NO: 20, an extension primer having a nucleic acid sequence represented by SEQ ID NO: 21, and a forward primer having a nucleic acid sequence represented by SEQ ID NO: 22, under the same conditions as described in the Examples above.

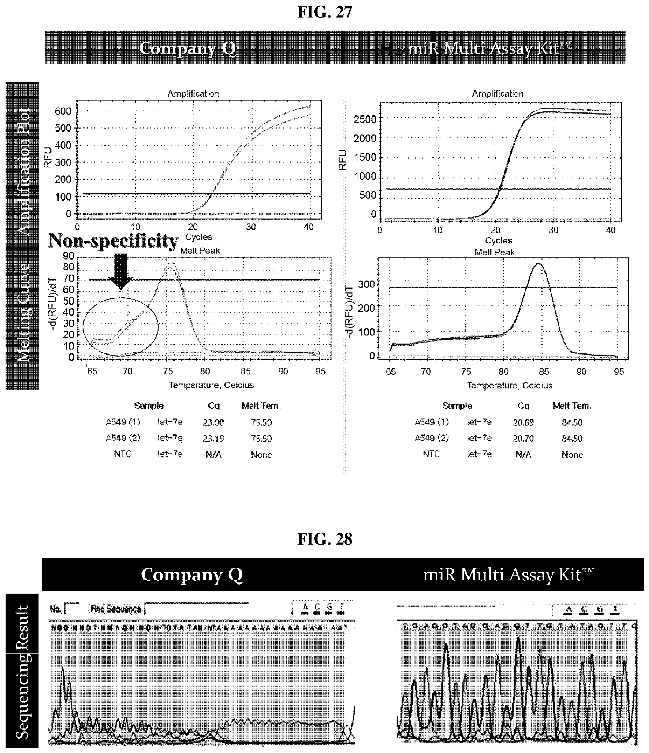

As a result, as shown in FIG. 27, a nonspecific amplification product was observed in the negative control when the Qiagen real-time PCR kit as the comparative example was used, whereas no nonspecific amplification was observed when the miRNA detection kit according to the embodiment of the present invention was used. In addition, as shown in FIG. 28, the results of sequencing the PCR amplification products indicated that the nucleotide sequence of the amplification product obtained using the Qiagen miRNA detection kit could not be properly confirmed, making it difficult to confirm whether or not the target miRNA would be properly amplified, whereas the amplification product obtained using the miRNA detection kit according to the embodiment of the present invention could be accurately sequenced.

### Experimental Example 2: Confirmation of Calibration Curve of PCR Amplification Products

In this experimental example, in order to examine whether or not the level of miRNA in a sample can be accurately quantified using the miRNA detection method according to one embodiment of the present invention, real-time PCR reaction was performed on serially diluted miRNA samples having predetermined concentrations, and then the detection threshold cycle (C_{q}) was measured.

Specifically, miR-145 was used as a target miRNA, and reverse transcription and PCR were performed under the same conditions as described in the Examples above.

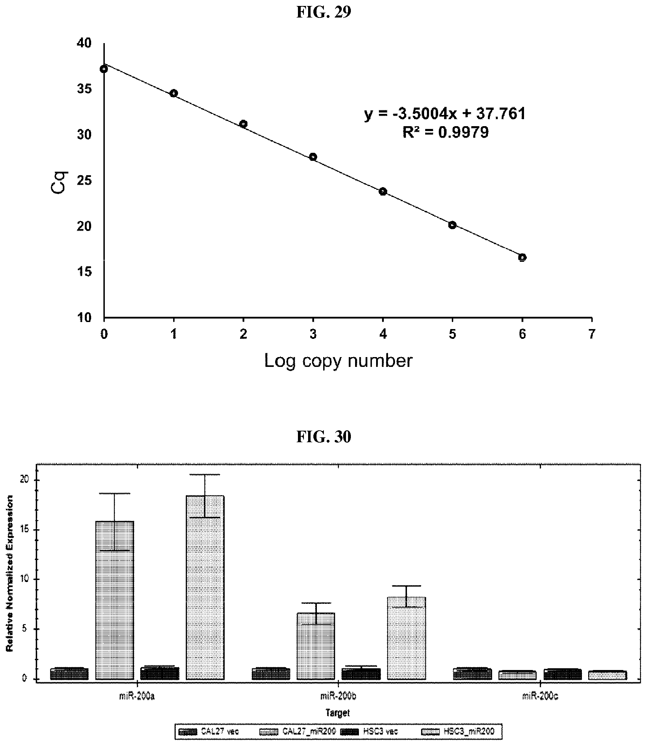

As a result, as shown in FIG. 29, it was confirmed that the C_{q} exhibited an accurate exponential relationship from very low concentrations, and the correlation between the log value of copy number of miRNA and the C_{q} was so high that its R² value reached 0.9979. These results suggest that the method of the present invention can be used not only for detection of miRNA, but also for accurate quantification of miRNA.

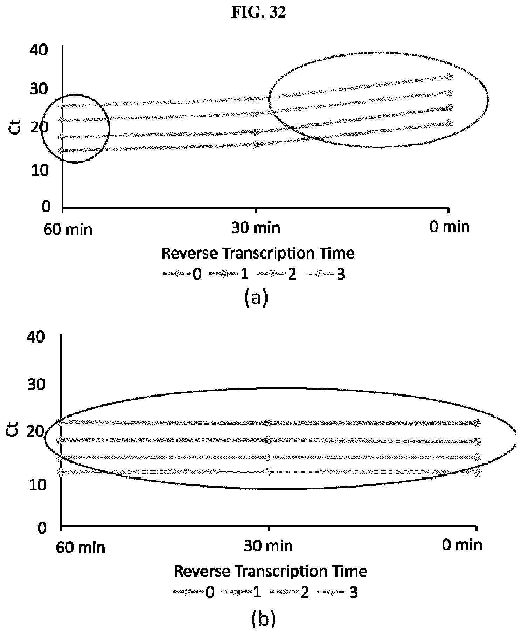

In addition, FIG. 32 is a graph showing a comparison between the Cₜ value obtained using the miRNA detection method according to one embodiment of the present invention and the Cₜ value obtained using the commercial miRNA detection kit (Qiagen) (real-time PCR results for human A549 lung cancer cell line). As can be seen in the graph of FIG. 32, the Cₜ value of the commercial miRNA detection kit (Qiagen) was changed and not stabilized in a short time, indicating that the reaction time of real-time PCR became longer and thus its reactivity decreased (see FIG. 32(a)), whereas the Cₜ value of the miRNA detection kit according to one embodiment of the present invention was stabilized after 10 minutes, indicating that the reaction time of real-time PCR became shorter and its reactivity increased (see FIG. 32(b)).

Therefore, according to the miRNA molecule detection method of the present invention, the reverse transcription reaction time is shortened by use of the short reverse transcription primer, and thus the overall PCR reaction rate can be increased, making it possible to quickly detect the miRNA molecule. In addition, according to the miRNA molecule detection method of the present invention, a template having a sufficient length for PCR reaction can be prepared by using the extension primer much longer than the reverse transcription primer in the primer extension step.

### Experimental Example 3: Classification of Subtypes of miRNA

For miRNAs, even same miRNAs show different subtypes with slightly different sequences depending on miRNA-derived individuals or cells. The present inventor examined whether or not the miRNA detection method according to one embodiment of the present invention could classify subtypes of miRNA.

Specifically, total RNA was isolated from CAL27 cells (ATCC CRL-2095) and HSC-3 cells by Trizol reagent, and then subjected to reverse transcription using reverse transcription primers (SEQ ID NOs: 23 to 25), each comprising at the 3'-end a first hybridizing oligonucleotide that binds complementarily to a 6-nt portion of the 3'-end of each of miR-200a, miR-200b and miR-200c and at the 5'-end a first adaptor oligonucleotide, at 42°C for 60 minutes. Next, 1 µL of each of the reverse transcription products was transferred into a reactor containing the real-time PCR reaction solution shown in Table 3 above, and then the enzyme was deactivated by heating at 70°C for 10 minutes. Using the BioRad CFX96 Touch Real-Time PCR system, primer extension and PCR was performed by melting the DNA at 95°C for 15 minutes, and then performing 40 cycles, each consisting of 95°C for 10 sec, and 60°C for 40 sec. Next, a melting curve was measured by heating at 95°C for 10 sec, and then heating from 65°C to 95°C at a rate of 0.5°C per 5 sec. The real-time PCR reaction was performed using each of extension primers (SEQ ID NOs: 26 to 28) specific for the respective miRNAs, and a universal forward primer (SEQ ID NO: 22) having a consensus sequence of the 5'-end of the extension primer. As a result, as shown in FIG. 30, it was confirmed that the CAL27 cells and the HSC3 cells all expressed miR-200a and miR-200b, but miR-200c was not detected in the two types of cells. These results are very similar to the results obtained using the ABI TaqMan^{®} MicroRNA Assay kit, and suggest that the method according to one embodiment of the present invention can provide reliable results.

### Experimental Example 4: Results according to whether or not reverse primer is added in real-time PCR reaction

In addition, the present inventor performed primer extension and real-time PCR in a single step, not in separate steps. The real-time PCR was performed using a primer pair composed of a forward primer having a nucleic acid sequence of the 5'-end of an extension primer, and a reverse primer which was the reverse transcription primer used in the reverse transcription. However, because the reverse primer was already used in the reverse transcription reaction (first step reaction) and remained in the reverse transcription reactant (first step reaction product) added to the primer extension and real-time PCR reactions (second step reaction), it was considered that even when the reverse primer was not separately added to the primer extension and real-time PCR reactions (second step reaction), the second step reaction would be normally performed. Accordingly, the examination was made of the effect of the following two conditions on real-time PCR: one condition where the reverse transcription primer is not added in the second step reaction; and the other condition wherein the reverse transcription primer is separately added in the second step reaction.

Specifically, as an RNA to be amplified, miR-16 was used, and as cells, the A549 lung cancer cell line was used. In case that the reverse transcription primer was not added in the second step reaction, reverse transcription, primer extension and real-time PCR reactions were performed under the conditions shown in Tables 2 and 3 above. In case that the reverse transcription primer was separately added in the second step reaction, the second step reaction was performed under the condition where 1 µL of the reverse transcription primer (2 µM) was added and distilled water was reduced by a volume of 1 µL. As the reverse transcription primer, an oligonucleotide having a nucleic acid sequence represented by SEQ ID NO: 29 was used, and as the miR-16-specific extension primer for primer extension and real-time PCR, an oligonucleotide having a nucleic acid sequence represented by SEQ ID NO: 30 was used, and as the forward primer, a universal forward primer having a nucleic acid sequence represented by SEQ ID NO: 22 was used. The reverse transcription step and the primer extension and real-time PCR step were performed under the same conditions as described in Experimental Example 2 above.

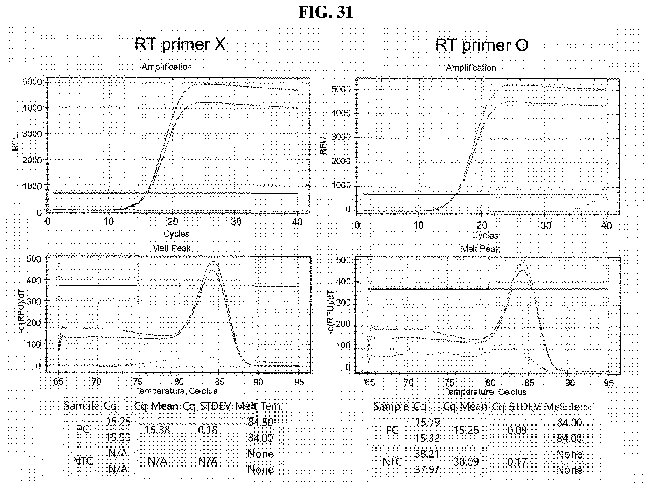

As a result, as shown in FIG. 31, when the reverse transcription primer was added in the second step reaction, a nonspecific reaction product was detected even in the negative control, but when the reverse transcription primer was not added in the second step reaction, this nonspecific reaction product was not detected. Therefore, it can be seen that more desirable results can be obtained when the reverse transcription primer is not added in the second step reaction.
## Claims

1. A method for detecting a miRNA molecule, comprising:
(a) reverse-transcribing a miRNA molecule using a reverse transcriptase and a short reverse primer for reverse transcription composed of a first hybridizing oligonucleotide and a first adaptor oligonucleotide, the first hybridizing oligonucleotide being composed of a nucleic acid sequence which hybridizes to a 3'-end portion of the miRNA molecule having 5 to 9 nt at the 3'-end of the miRNA molecule, and the first adaptor oligonucleotide being attached to the 5'-end of the first hybridizing oligonucleotide and being any nucleic acid sequence which does not hybridize to the miRNA molecule;
(b) deactivating the reverse transcriptase and melting a DNA produced by the reverse transcription, thereby preparing a single-stranded cDNA reverse-transcribed from the miRNA molecule; and
(c) transferring a portion or all of the reverse-transcription reactant into a reactor for primer extension and PCR reactions containing an extension primer longer than the reverse primer for reverse transcription, a forward primer, and a heat-stable DNA polymerase, the extension primer being composed of a second hybridizing oligonucleotide and a second adaptor oligonucleotide, the second hybridizing oligonucleotide being capable of hybridizing specifically to a portion of the single-stranded cDNA reverse-transcribed from the miRNA molecule, the portion of the single-stranded cDNA excluding a portion corresponding to the 3'-end portion of the miRNA molecule, and the second adaptor oligonucleotide being attached to the 5'-end of the second hybridizing oligonucleotide and having any nucleic acid sequence which does not hybridize to the single-stranded cDNA, and the forward primer having a sequence within the second adaptor oligonucleotide, and then performing primer extension and PCR reactions in a single step;
wherein a reverse primer is not added in the (c);
wherein the short reverse primer for reverse transcription used in the reverse transcription of the (a) also functions as a reverse primer in the PCR reaction, and thus the short reverse primer for reverse transcription contained in the reverse transcription reactant is used as a reverse primer in the PCR reaction;
wherein the short reverse transcription primer is 18 to 28 nt in length; and
wherein the second adaptor oligonucleotide is 56 to 63 nt in length.

2. The method of claim 1, wherein the second adaptor oligonucleotide comprises either a miRNA -tagging oligonucleotide dependent on the kind of miRNA molecule to be amplified, or the miRNA -tagging oligonucleotide and a consensus oligonucleotide having a consensus nucleic acid sequence independent of the kind of miRNA to be amplified.

## Patentansprüche

1. Verfahren zur Detektion eines miRNA-Moleküls, umfassend:
(a) Rückwärts-Transkribieren eines miRNA-Moleküls unter Verwendung einer Rückwärts-Transkriptase und eines kurzen Rückwärts-Primers für eine Rückwärts-Transkription, bestehend aus einem ersten hybridisierenden Oligonukleotid und einem ersten Adapter-Oligonukleotid, wobei das erste hybridisierende Oligonukleotid aus einer Nukleinsäuresequenz besteht, die an einen 3'-Endabschnitt des miRNA-Moleküls mit 5 bis 9 nt am 3'-Ende des miRNA-Moleküls hybridisiert, und wobei das erste Adapter-Oligonukleotid am 5'-Ende des ersten hybridisierenden Oligonukleotids angebracht ist und eine Nukleinsäuresequenz ist, die nicht an das miRNA-Molekül hybridisiert;
(b) Deaktivieren der Rückwärts-Transkriptase und Schmelzen einer durch die Rückwärts-Transkription erzeugten DNA, wodurch eine einzelsträngige, aus dem miRNA-Molekül rückwärts transkribierte cDNA erzeugt wird; und
(c) Übertragen eines Teils oder des gesamten Rückwärts-Transkriptions-Reaktanten in einen Reaktor für die Primerverlängerung und PCR-Reaktionen, die einen Verlängerungsprimer, der länger ist als der Rückwärts-Primer für die Rückwärts-Transkription, einen Vorwärts-Primer und eine hitzestabile DNA-Polymerase enthalten, wobei der Verlängerungsprimer aus einem zweiten hybridisierenden Oligonukleotid und einem zweiten Adapter-Oligonukleotid besteht, wobei das zweite hybridisierende Oligonukleotid in der Lage ist, spezifisch an einen Teil der einzelsträngigen, aus dem miRNA-Molekül rückwärts transkribierten cDNA zu hybridisieren, wobei der Teil der einzelsträngigen cDNA einen dem 3'-Endabschnitt des miRNA-Moleküls entsprechenden Teil ausschließt, und wobei das zweite Adapter-Oligonukleotid an dem 5'-Ende des zweiten hybridisierenden Oligonukleotids angebracht ist und eine Nukleinsäuresequenz aufweist, die nicht an die einzelsträngige cDNA hybridisiert, und wobei der Vorwärts-Primer eine Sequenz innerhalb des zweiten Adapter-Oligonukleotids aufweist, und dann Ausführen der Primerverlängerung und der PCR-Reaktionen in einem einzigen Schritt;
wobei ein Rückwärts-Primer in (c) nicht zugefügt wird; wobei der Rückwärts-Primer für die Rückwärts-Transkription, der bei der Rückwärts-Transkription von (a) verwendet wird, auch als Rückwärts-Primer in der PCR-Reaktion wirkt, und somit der kurze Rückwärts-Primer für die Rückwärts-Transkription, der in dem Rückwärts-Transkriptions-Reaktanten enthalten ist, als Rückwärts-Primer in der PCR-Reaktion verwendet wird;
wobei der kurze Rückwärts-Transkriptions-Primer eine Länge von 18 bis 28 nt aufweist; und
wobei das zweite Adapter-Oligonukleotid eine Länge von 56 bis 63 nt aufweist.

2. Verfahren nach Anspruch 1, wobei entweder das zweite Adapter-Oligonukleotid ein miRNA-Markierungs-Oligonukleotid abhängig von der Art des zu amplifizierenden miRNA-Moleküls umfasst oder das miRNA-Markierungs-Oligonukleotid und ein Konsensus-Oligonukleotid eine Konsensus-Nukleinsäuresequenz unabhängig von der Art der zu amplifizierenden miRNA aufweist.

## Revendications

1. Procédé de détection d'une molécule d'miARN, comprenant :
(a) la transcription inverse d'une molécule d'miARN en utilisant une transcriptase inverse et une courte amorce inverse pour la transcription inverse composée d'un premier oligonucléotide d'hybridation et d'un premier oligonucléotide adaptateur, le premier oligonucléotide d'hybridation étant composé d'une séquence d'acide nucléique qui s'hybride à une partie 3'-terminale de la molécule d'miARN ayant 5 à 9 nt à l'extrémité 3' de la molécule d'miARN et le premier oligonucléotide adaptateur étant attaché à l'extrémité 5' du premier oligonucléotide d'hybridation et étant une quelconque séquence d'acide nucléique qui ne s'hybride pas à la molécule d'miARN ;
(b) la désactivation de la transcriptase inverse et la fusion d'un ADN produit par la transcription inverse, préparant ainsi un ADNc simple brin transcrit en inverse à partir de la molécule d'miARN ; et
(c) le transfert d'une partie ou de l'ensemble du réactif de transcription inverse dans un réacteur pour extension d'amorce et des réactions de PCR contenant une amorce d'extension plus longue que l'amorce inverse pour la transcription inverse, une amorce sens et une ADN polymérase thermostable, l'amorce d'extension étant composée d'un second oligonucléotide d'hybridation et d'un second oligonucléotide adaptateur, le second oligonucléotide d'hybridation étant capable de s'hybrider spécifiquement à une partie de l'ADNc simple brin transcrit en inverse de la molécule d'miARN, la partie de l'ADNc simple brin excluant une partie correspondant à la partie d'extrémité 3' de la molécule d'miARN et le second oligonucléotide adaptateur étant attaché à l'extrémité 5' du second oligonucléotide d'hybridation et ayant une quelconque séquence d'acide nucléique qui ne s'hybride pas à l'ADNc simple brin et l'amorce sens ayant une séquence dans le second oligonucléotide adaptateur puis effectuant l'extension d'amorce et les réactions de PCR en une seule étape ;
une amorce inverse n'étant pas ajoutée dans le (c) ; l'amorce inverse courte pour la transcription inverse utilisée dans la transcription inverse du (a) fonctionnant aussi comme une amorce inverse dans la réaction de PCR et ainsi la courte amorce inverse pour la transcription inverse contenue dans le réactif de transcription inverse est utilisée comme une amorce inverse dans la réaction de PCR ;
la courte amorce de transcription inverse étant de 18 à 28 nt de long; et
le second oligonucléotide adaptateur étant de 56 à 63 nt de long.

2. Procédé selon la revendication 1, dans lequel le second oligonucléotide adaptateur comprend un oligonucléotide d'étiquetage d'miARN dépendant du type de molécule d'mi-ARN à amplifier ou l'oligonucléotide d'étiquetage d'miARN et un oligonucléotide consensus ayant une séquence d'acide nucléique consensus indépendante du type d'miARN à amplifier.


<110> HeimBiotek Inc.
<120> A kit and method for detecting RNA molecules
<130> OP17-0008PCT
<150> PCT/KR 2016/007971
   <151> 2016-07-21
<160> 30
<170> KoPatentIn 3.0
<210> 1
   <211> 29
   <212> DNA
   <213> Artificial Sequence
<220>
   <223> primer for reversetranscription with poly A tailing
<400> 1
   gcaccaccac tgattagttt ttttttttt 29
<210> 2
   <211> 18
   <212> DNA
   <213> Artificial Sequence
<220>
   <223> primer for the reversetranscription and reverse primer for PCR of hsa-miR-532
<400> 2
   gcgagcatgc agacggtc 18
<210> 3
   <211> 20
   <212> DNA
   <213> Artificial Sequence
<220>
   <223> primer for the reversetranscription and reverse primer for PCR of hsa-miR-196b
<400> 3
   gctagtcatg ccagcccaac 20
<210> 4
   <211> 19
   <212> DNA
   <213> Artificial Sequence
<220>
   <223> primer for the reversetranscription and reverse primer for PCR of hsa-miR-362
<400> 4
   gcgagcatcg cagactcac 19
<210> 5
   <211> 19
   <212> DNA
   <213> Artificial Sequence
<220>
   <223> primer for the reversetranscription and reverse primer for PCR of hsa-miR-29c
<400> 5
   gcgagcatcg cagtaaccg 19
<210> 6
   <211> 19
   <212> DNA
   <213> Artificial Sequence
<220>
   <223> primer for the reversetranscription and reverse primer for PCR of hsa-miR-106b
<400> 6
   gcgagcactg cacatctgc 19
<210> 7
   <211> 20
   <212> DNA
   <213> Artificial Sequence
<220>
   <223> primer for the reversetranscription and reverse primer for PCR of hsa-miR-200c
<400> 7
   gcgagcactt cacgtccatc 20
<210> 8
   <211> 18
   <212> DNA
   <213> Artificial Sequence
<220>
   <223> primer for the reversetranscription and reverse primer for PCR of hsa-miR-127
<400> 8
   gcgacgatgc agagccaa 18
<210> 9
   <211> 20
   <212> DNA
   <213> Artificial Sequence
<220>
   <223> primer for the reversetranscription and reverse primer for PCR of hsa-miR-145
<400> 9
   gcgagtccgc atagagggat 20
<210> 10
   <211> 77
   <212> DNA
   <213> Artificial Sequence
<220>
   <223> extension primer for hsa-miR-532
<400> 10
   gcacctccag gaccaatctt gtagccagga tcttgccatc ctatggaact gcctcggtga 60
   gcatgccttg agtgtag 77
<210> 11
   <211> 77
   <212> DNA
   <213> Artificial Sequence
<220>
   <223> extension primer for hsa-miR-196b
<400> 11
   gcacctccag gaccaatctt gtagccagga tcttgccatc ctatggaact gcctcggtga 60
   gtaggtagtt tcctgtt 77
<210> 12
   <211> 79
   <212> DNA
   <213> Artificial Sequence
<220>
   <223> extension primer for hsa-miR-362
<400> 12
   gcacctccag gaccaatctt gtagccagga tcttgccatc ctatggaact gcctcggtga 60
   gaatccttgg aacctaggt 79
<210> 13
   <211> 77
   <212> DNA
   <213> Artificial Sequence
<220>
   <223> extension primer for hsa-miR-29c
<400> 13
   gcacctccag gaccaatctt gtagccagga tcttgccatc ctatggaact gcctcggtga 60
   gtagcaccat ttgaaat 77
<210> 14
   <211> 76
   <212> DNA
   <213> Artificial Sequence
<220>
   <223> extension primer for hsa-miR-106b
<400> 14
   gcacctccag gaccaatctt gtagccagga tcttgccatc ctatggaact gcctcggtga 60
   gtaaagtgct gacagt 76
<210> 15
   <211> 78
   <212> DNA
   <213> Artificial Sequence
<220>
   <223> extension primer for hsa-miR-200c
<400> 15
   gcacctccag gaccaatctt gtagccagga tcttgccatc ctatggaact gcctcggtga 60
   gtaatactgc cgggtaat 78
<210> 16
   <211> 77
   <212> DNA
   <213> Artificial Sequence
<220>
   <223> extension primer for hsa-miR-127
<400> 16
   gcacctccag gaccaatctt gtagccagga tcttgccatc ctatggaact gcctcggtga 60
   gtcggatccg tctgagc 77
<210> 17
   <211> 78
   <212> DNA
   <213> Artificial Sequence
<220>
   <223> extension primer for hsa-miR-145
<400> 17
   gcacctccag gaccaatctt gtagccagga tcttgccatc ctatggaact gcctcggtga 60
   ggtccagttt tcccagga 78
<210> 18
   <211> 18
   <212> DNA
   <213> Artificial Sequence
<220>
   <223> universal forward primer for poly A tailed miRNA
<400> 18
   gcacctccag gaccaatc 18
<210> 19
   <211> 17
   <212> DNA
   <213> Artificial Sequence
<220>
   <223> universal reverse primer for poly A tailed miRNA
<400> 19
   gcaccaccac tgattag 17
<210> 20
   <211> 21
   <212> DNA
   <213> Artificial Sequence
<220>
   <223> RT primer for hsa-let-7e-5p
<400> 20
   tgcagggtgg cagccaacta t 21
<210> 21
   <211> 72
   <212> DNA
   <213> Artificial Sequence
<220>
   <223> extension primer for hsa-let-7e-5p
<400> 21
   taagttccgc tgtgtgccgc atctcaccgg gcggcgcttt gagcacggtg tgacggtgag 60
   gtaggaggtt gt 72
<210> 22
   <211> 20
   <212> DNA
   <213> Artificial Sequence
<220>
   <223> universal foward primer
<400> 22
   taagttccgc tgtgtgccgc 20
<210> 23
   <211> 21
   <212> DNA
   <213> Artificial Sequence
<220>
   <223> RT primer for miR-200a
<400> 23
   tgcagggtgg cagccgctac a 21
<210> 24
   <211> 21
   <212> DNA
   <213> Artificial Sequence
<220>
   <223> RT primer for miR-200b
<400> 24
   tgcagggtgg cagccactac t 21
<210> 25
   <211> 21
   <212> DNA
   <213> Artificial Sequence
<220>
   <223> RT primer for miR-200c
<400> 25
   tgcagggtgg cagccctacc t 21
<210> 26
   <211> 72
   <212> DNA
   <213> Artificial Sequence
<220>
   <223> extension primer for miR-200a
<400> 26
   taagttccgc tgtgtgccgc atctcaccgg gcggcgcttt gagcacggtg tgacggtaac 60
   actgtctggt aa 72
<210> 27
   <211> 72
   <212> DNA
   <213> Artificial Sequence
<220>
   <223> extension primer for miR-200b
<400> 27
   taagttccgc tgtgtgccgc atctcaccgg gcggcgcttt gagcacggtg tgacggtaat 60
   actgcctggt aa 72
<210> 28
   <211> 73
   <212> DNA
   <213> Artificial Sequence
<220>
   <223> extension primer for miR-200c
<400> 28
   taagttccgc tgtgtgccgc atctcaccgg gcggcgcttt gagcacggtg tgacggtaat 60
   actgccgggt aat 73
<210> 29
   <211> 21
   <212> DNA
   <213> Artificial Sequence
<220>
   <223> RT primer for miR-16
<400> 29
   tgcagggtgg cagcccgcca a 21
<210> 30
   <211> 72
   <212> DNA
   <213> Artificial Sequence
<220>
   <223> extension primer for miR-16
<400> 30
   taagttccgc tgtgtgccgc atctcaccgg gcggcgcttt gagcacggtg tgacggtagc 60
   agcacgtaaa ta 72